# EUROPÄISCHE PATENTSCHRIFT

(11) **EP 2 771 330 B1**
(45) Veröffentlichungstag und Bekanntmachung des Hinweises auf die Patenterteilung: **30.12.2015**
(21) Anmeldenummer: 12775711.0
(22) Anmeldetag: 26.10.2012
(51) Int. Cl.: C07D 309/04

(54) **VERFAHREN ZUR HERSTELLUNG VON CIS-ROSENOXID**
METHOD FOR PRODUCING CIS-ROSE OXIDE
PROCEDE DE PREPARATION D'OXYDE DE ROSE CIS

(30) Priorität: 28.10.2011 EP 11187106
(43) Veröffentlichungstag der Anmeldung: 03.09.2014
(73) Patentinhaber: BASF SE, 67056 Ludwigshafen (DE)
(72) Erfinder: HEIDEMANN, Thomas, 68519 Viernheim (DE); KÖNIGSMANN, Lucia, 70197 Suttgart (DE)
(86) Internationale Anmeldenummer: PCT/EP2012/071195
(87) Internationale Veröffentlichungsnummer: WO 2013/060805

(56) Entgegenhaltungen:
- WO-A1-2009/077550
- TROST, B. M. ET AL.: "Sequential Ru-Pd Catalysis. A Two-Catalyst One-Pot Protocol for the Synthesis of N- and O-Heterocycles", JOURNAL OF THE AMERICAN CHEMICAL SOCIETY, Bd. 128, 2006, Seiten 6745-6754, XP002665164, AMERICAN CHEMICAL SOCIETY ISSN: 0002-7863

## Beschreibung

Die vorliegende Erfindung betrifft ein Verfahren zur Herstellung einer an cis-2-(2-Methyl-prop-1-en-yl)-4-methyl-tetrahydropyran angereicherten Zusammensetzung, umfassend die katalytische Hydrierung von 2-(2-Methyl-prop-1-en-yl)-4-methylen-tetrahydropyran in Gegenwart von Wasserstoff und eines heterogenen rutheniumhaltigen Katalysators.

Cis-2-(2-Methyl-prop-1-en-yl)-4-methyl-tetrahydropyran stellt eine wertvolle, auch als cis-Rosenoxid bezeichnete Aromachemikalie dar. Sie fällt üblicherweise in Form eines Diastereomerengemischs mit der entsprechenden trans-konfigurierten Verbindung an, wobei sich die cis-konfigurierte Verbindung als die wertvollere, weil besser duftende Verbindung erwiesen hat. Da sich die Diastereomere, insbesondere bei Herstellung im technischen Maßstab, nur schwer voneinander trennen lassen, besteht ein nachhaltiger Bedarf an Herstellverfahren, bei denen in hoher Ausbeute möglichst selektiv das bevorzugte cis-Isomer des Rosenoxids gebildet wird.

J. H. P. Tyman and B. J. Willis beschreiben in Tetrahedron Letters No. 51, 4507 - 4508, 1970, die säurekatalysierte Umsetzung von 3-Alken-1-olen mit Aldehyden, speziell die Umsetzung von 3-Methyl-2-buten-1-al mit 2-Methyl-1-buten-4-ol und anschließender Dehydratisierung. Das so erhaltene, eine exocyclische Methylengruppe aufweisende Zwischenprodukt wurde homogenkatalytisch in Gegenwart von SnCl₂/H₂PtCl₆ zum racemischen cis-2-(2-Methyl-prop-1-en-yl)-4-methyl-tetrahydropyran hydriert.

WO 79/00509 offenbart ein Verfahren zur Herstellung von bezüglich des cis-Isomeren angereicherten Gemischen von cis- und trans-(2-Methyl-prop-1-en-yl)-4-methyl-tetrahydropyran durch katalytische Hydrierung der entsprechenden, in 4-Position eine exo-Methylengruppe aufweisenden Vorstufe. Als geeignete Hydrierkatalysatoren werden Raney-Nickel und Palladiumkatalysatoren, speziell Palladium auf Kohlenstoff, genannt. Die Isomerenanreicherung wird durch Behandlung des Hydrierproduktes mit einem sauren oder Lewis-sauren Reagenz erreicht. Als bevorzugte Lewis-Säure wird Bortrifluorid genannt. Die beispielhaft beschriebene Hydrierung mit Raney-Nickel mit anschließender Destillation liefert in einer Ausbeute von 87,9 % der Theorie ein Gemisch der cis- und trans-Isomere in einem Verhältnis von 4 : 6. Dieses Gemisch wird in der anschließenden Isomerisierung in einer Ausbeute von 86,5 % der Theorie in ein Isomerengemisch im Verhältnis von etwa 85 : 15 umgesetzt.

Die EP 0 082 401 A1 offenbart ein Verfahren zur Herstellung von überwiegend, d. h. zu mindestens 85 %, das cis-Isomere enthaltendem Rosenoxid. Das Verfahren ist dadurch gekennzeichnet, dass man 2-(2-Methyl-prop-1-en-yl)-4-methylen-tetrahydropyran ("Dehydrorosenoxid") an einem Platindioxid- oder einem Platin/Kohle-Katalysator in Gegenwart eines stark sauren Kationenaustauschers hydriert. Bei der beispielhaft beschriebenen isomerisierenden Hydrierung von Dehydrorosenoxid zu Rosenoxid wird eine Ausbeute von bis zu 87 % d. Th. mit einem Isomerengehalt des trans-Isomeren von 90,5 % bzw. des cis-Isomeren von 7 % erreicht.

Die WO 2009/077550 beschreibt ein Verfahren zur Herstellung von cis-2-(2-Methyl-prop-1-en-yl)-4-methyl-tetrahydropyran, umfassend die katalytische Hydrierung von 2-(2-Methyl-prop-1-en-yl)-4-methylen-tetrahydropyran in Gegenwart von Wasserstoff und eines heterogenen Katalysators, der Ruthenium auf einem Kohlenstoffträger umfasst und anschließende Isomerisierung zur Erhöhung des Anteils an cis-Isomer durch Inkontaktbringen der so erhaltenen Verbindungen mit einem stark sauren Kationenaustauscher. Obwohl dieses Verfahren die Herstellung von Rosenoxid mit guten Umsätzen und Selektivitäten erlaubt, ist eine weitere Verbesserung der katalytischen Hydrierung wünschenswert.

Überraschenderweise wurde gefunden, dass durch den Einsatz eines heterogenen Katalysators, der Ruthenium auf einem Aluminiumoxidträger umfasst, sich die Selektivität der Hydrierung noch einmal signifikant verbessern lässt. Der neu verwendete Katalysator lässt sich zudem einer einfacheren Aktivierung unterziehen, bei der zudem eine verbesserte Aktivität erreicht wird.

Gegenstand der Erfindung ist daher ein Verfahren zur Herstellung einer an cis-2-(2-Methyl-prop-1-en-yl)-4-methyl-tetrahydropyran angereicherten Zusammensetzung, umfassend die katalytische Hydrierung von 2-(2-Methyl-prop-1-en-yl)-4-methylen-tetrahydropyran in Gegenwart von Wasserstoff und eines heterogenen Katalysators, der Ruthenium auf einem Aluminiumoxidträger umfasst.

Die Bezeichnung "Rosenoxid" bezeichnet im Rahmen der Erfindung cis/trans-Gemische jedweder Zusammensetzung sowie die reinen Konformation-Isomere. Die Bezeichnung "Rosenoxid" bezeichnet weiterhin alle Enantiomere in Reinform sowie racemische und optisch aktive Gemische der Enantiomeren des Rosenoxids.

In einer bevorzugten Ausführungsform betrifft das erfindungsgemäße Verfahren ein Verfahren zur Herstellung eines Isomerengemisches von cis-2-(2-Methyl-prop1-en-yl)-4-methyl-tetrahydropyran der Formel (IIa) und trans-2-(2-Methyl-prop-1-en-yl)-4-methyl-tetrahydropyran der Formel (IIb).

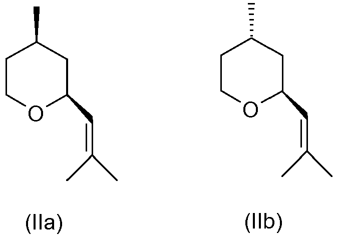

Die Verbindungen der Formeln (IIa) und (IIb) fallen erfindungsgemäß in racemischer Form an. Die Formelbilder (IIa) und (IIb) dienen dementsprechend der Veranschaulichung der relativen Konfiguration der beiden Stereozentren und stehen jeweils für die racemischen Gemische der jeweiligen Enantiomerenpaare.

Das als Ausgangsstoff eingesetzte 2-(2-Methyl-prop-1-en-yl)-4-methylen-tetrahydropyran kann im Prinzip auf jedwede Weise hergestellt sein, und an seine Beschaffenheit oder Reinheit werden keine speziellen Anforderungen gestellt, die über den Rahmen des für synthetische Zwecke Üblichen hinausgehen. Vorzugsweise wird racemisches 2-(2-Methyl-prop-1-en-yl)-4-methylen-tetrahydropyran eingesetzt, wie es von der Formel (I) wiedergegeben wird.

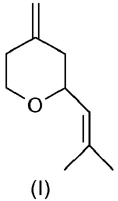

Im einer bevorzugten Ausführungsform umfasst das erfindungsgemäße Verfahren als zusätzlichen, vorgeschalteten Verfahrensschritt die Bereitstellung von 2-(2-Methylprop-1-en-yl)-4-methylen-tetrahydropyran (Dehydrorosenoxid) der Formel (I) durch Umsetzung von 3-Methyl-but-3-en-1-ol (Isoprenol) der Formel (III) 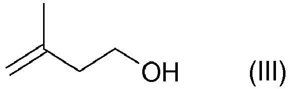 mit 3-Methyl-but-2-en-1-al (Prenal) der Formel (IV) 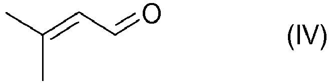 in einer Kondensationsreaktion. Details können im Folgenden der Beschreibung zu Schritt i) entnommen werden.

Unter einer an cis-2-(2-Methylprop-1-en-yl)-4-methyl-tetrahydropyran angereicherten Zusammensetzung wird im Rahmen der Erfindung eine Zusammensetzung verstanden, die mehr als 50 Gew.-% an cis-2-(2-Methylprop-1-en-yl)-4-methyl-tetrahydropyran, bezogen auf die Gesamtmenge an cis- und trans-Isomeren in der Zusammensetzung, enthält.

Im Allgemeinen gelingt mit dem erfindungsgemäßen Verfahren die Herstellung von an cis-2-(2-Methylprop-1-en-yl)-4-methyl-tetrahydropyran angereicherten Zusammensetzungen, die vorzugsweise wenigstens 70 Gew.-%, besonders bevorzugt wenigstens 80 Gew.-%, insbesondere wenigstens 90 Gew.-% an cis-2-(2-Methylprop-1-en-yl)-4-methyl-tetrahydropyran, bezogen auf die Gesamtmenge an cis- und trans-Isomeren in der Zusammensetzung, enthalten. Entsprechend enthält die nach dem erfindungsgemäßen Verfahren erhaltene Zusammensetzung vorzugsweise höchstens 30 Gew.-%, besonders bevorzugt höchstens 20 Gew.-%, insbesondere höchstens 10 Gew.-% an trans-2-(2-Methylprop-1-en-yl)-4-methyl-tetrahydropyran, bezogen auf die Gesamtmenge an cis- und trans-Isomeren in der Zusammensetzung. In einer speziellen Ausführung enthält die nach dem erfindungsgemäßen Verfahren erhaltene Zusammensetzung 90 bis 98 % cis-2-(2-Methyl-prop-1-en-yl)-4-methyl-tetrahydropyran und 2 bis 10 % Gew.-% trans-2-(2-Methyl-prop-1-en-yl)-4-methyl-tetrahydropyran, jeweils bezogen auf die Gesamtmenge an cis- und trans-Isomeren in der Zusammensetzung.

Bevorzugt enthält die nach dem erfindungsgemäßen Verfahren erhaltene, an cis-2-(2-Methylprop-1-en-yl)-4-methyl-tetrahydropyran angereicherte Zusammensetzung höchstens 10 Gew.-%, besonders bevorzugt höchstens 5 Gew.-%, insbesondere höchstens 2 Gew.-% an von cis- und trans-2-(2-Methylprop-1-en-yl)-4-methyl-tetrahydropyran verschiedenen Verbindungen.

In dem erfindungsgemäßen Verfahren wird ein heterogener Katalysator eingesetzt, der Ruthenium auf einem Aluminiumoxidträger umfasst.

Unter einem Aluminiumoxidträger wird im Rahmen der Erfindung ein Träger verstanden, der zu wenigstens 50 Gew.-%, bezogen auf das Gesamtgewicht des Trägermaterials, aus Aluminiumoxid besteht.

Der erfindungsgemäß eingesetzte Träger kann Aluminiumoxid im Gemisch mit weiteren Trägermaterialien enthalten. Geeignete weitere Trägermaterialien sind beispielsweise ausgewählt unter Graphit, Siliziumdioxid, Titandioxid, Zirkondioxid und Mischungen davon. Bevorzugt als weitere Trägermaterialien sind Titandioxid und/oder Zirkondioxid. Vorzugsweise besteht der Träger zu wenigstens 80 Gew.-%, besonders bevorzugt zu wenigstens 90 Gew.-%, insbesondere zu mindestens 96 Gew.-%, bezogen auf das Gesamtgewicht des Trägermaterials, aus Aluminiumoxid.

Besonders bevorzugt für den Einsatz in dem erfindungsgemäßen Verfahren ist ein Katalysator, bei dem der Aluminiumoxidanteil des Trägers im Wesentlichen aus alpha-Aluminiumoxid besteht.

Der Aluminiumoxidanteil des Trägers besteht bevorzugt zu mindestens 80 Gew.-%, besonders bevorzugt zu mindestens 90 Gew.-%, insbesondere zu mindestens 98 Gew.-% aus alpha-Aluminiumoxid, bezogen auf den gesamten Aluminiumoxidanteil des Trägers. Die Phasenzusammensetzung des Trägers kann mit XRD (X-Ray Diffraction) bestimmt werden.

Der erfindungsgemäß eingesetzte Katalysator enthält Ruthenium als Aktivmetall. Bevorzugt enthält der Katalysator 0,001 bis 10 Gew.-% Ruthenium, besonders bevorzugt 0,01 bis 5 Gew.-% Ruthenium, insbesondere 1 bis 3 Gew.-% Ruthenium, bezogen auf das Gesamtgewicht des Katalysators.

Die Gewichtsangaben bezüglich Ruthenium und weiterer Metalle beziehen sich auf das Gewicht des Metalls, auch wenn die Metalle in der Regel in oxidischer Form auf dem Träger vorliegen.

Zusätzlich kann der erfindungsgemäße Katalysator wenigstens ein weiteres, von Ruthenium verschiedenes Metall als Aktivmetall oder als Promotor enthalten. Weitere Aktivmetalle sind vorzugsweise ausgewählt sind aus den Elementen der Gruppen 7 bis 11 des Periodensystems der Elemente.

Die erfindungsgemäßen Katalysatoren können zusätzlich wenigstens ein weiteres Metall enthalten, vorzugsweise ausgewählt unter Kupfer, Gold, Palladium, Platin, Osmium, Iridium, Silber, Rhenium und Mischungen davon. Bevorzugt enthält der Katalysator 0 bis 10 Gew.-%, bevorzugt 0,001 bis 5 Gew.-%, speziell 0,01 bis 1 Gew.-% eines weiteren Metalls, ausgewählt unter Kupfer, Gold, Palladium, Platin, Osmium, Iridium, Silber, Rhenium und Mischungen davon, bezogen auf das Gesamtgewicht des Katalysators.

Bevorzugte weitere Metalle sind Kupfer und/oder Gold.

Die Katalysatoren können ferner ein weiteres Metall oder mehrere weitere Metalle als Promotoren enthalten. Die Promotoren sind vorzugsweise ausgewählt unter Alkalimetallen, Erdalkalimetallen, Seltenerdmetallen, Titan, Mangan, Molybdän, Zinn und Mischungen davon. Diese sind üblicherweise in einer Menge von 0 bis 10 Gew.-%, bezogen auf das Gesamtgewicht des Katalysators, enthalten. Sofern die Katalysatoren ein weiteres Metall oder mehrere weitere Metalle als Promotoren enthalten, so sind diese vorzugsweise in einer Menge von 0,001 bis 5 Gew.-%, besonders bevorzugt in einer Menge von 0,1 bis 3 Gew.-%, bezogen auf das Gesamtgewicht des Katalysators, enthalten.

Die Promotoren sind besonders bevorzugt ausgewählt unter Lithium, Natrium, Kalium, Rubidium, Cäsium, Magnesium, Calcium, Strontium, Barium, Scandium, Yttrium, Lanthan, Cer, Praseodym, Neodym, Titan, Mangan, Molybdän, Zinn und Mischungen davon.

Die Promotoren sind insbesondere ausgewählt unter Lithium, Natrium, Kalium, Magnesium, Calcium, Scandium, Yttrium, Lanthan, Cer, Titan, Mangan, Molybdän, Zinn und Mischungen davon.

Die Promotoren sind speziell ausgewählt unter Kalium, Magnesium, Lanthan, Cer, Titan, Mangan, Molybdän, Zinn und Mischungen davon.

Bevorzugte erfindungsgemäße Katalysatoren enthalten
a) 0,001 bis 10 Gew.-%, bevorzugt 1 bis 3 Gew.-% Ruthenium,
b) 0 bis 5 Gew.-%, bevorzugt 0 bis 3 Gew.-% eines oder mehrerer Erdalkalimetalle,
c) 0 bis 5 Gew.-%, bevorzugt 0 bis 3 Gew.-% eines oder mehrerer Alkalimetalle,
d) 0 bis 10 Gew.-%, bevorzugt 0 bis 3 Gew.-% eines oder mehrerer Seltenerdmetalle,
e) 0 bis 10 Gew.-%, bevorzugt 0 bis 1 Gew.-% eines oder mehrerer weiterer Metalle, ausgewählt aus der Gruppe bestehend aus Kupfer, Gold, Palladium, Platin, Osmium, Iridium, Silber und Rhenium,
jeweils bezogen auf das Gesamtgewicht des Katalysators. Die Gewichtsangaben beziehen sich auf das Gewicht des Metalls, auch wenn die Metalle in der Regel in oxidischer Form auf dem Träger vorliegen.

Ganz besonders bevorzugt als Aktivmetall ist Ruthenium, das im Allgemeinen in Mengen von 0,001 bis 10 Gew.-%, bezogen auf das Gewicht des Katalysators, enthalten ist. In einer speziellen Ausführungsform enthält der erfindungsgemäße Katalysator 1 bis 3 Gew.- %, beispielsweise etwa 1,6 Gew.-%, Ruthenium auf alpha-Aluminiumoxid als Träger und daneben keine weiteren Aktivmetalle und Promotormetalle. Bevorzugt liegt das Ruthenium als RuO₂ vor.

Die erfindungsgemäßen Katalysatoren können durch übliche, dem Fachmann bekannte Verfahren hergestellt werden. In einer bevorzugten Ausführung werden die Katalysatoren durch Tränkung des Trägermaterials mit wässrigen Lösungen von Salzen der Metalle erhalten. Von Gold verschiedene Metalle werden üblicherweise als wässrige Lösungen ihrer Chloride, Oxichloride oder Oxide auf den Träger aufgebracht. Die Rutheniumkatalysatoren können beispielsweise durch Tränkung des Trägermaterials mit wässrigen Lösungen von RuCl₃, gegebenenfalls dem Salz wenigstens eines weiteren Aktivmetalls und/oder gegebenenfalls eines Promotors zur Dotierung erhalten werden. Bevorzugt werden jeweils die Chloride eingesetzt. Die Formgebung des Katalysators kann nach oder bevorzugt vor der Tränkung des Trägermaterials erfolgen.

Die erfindungsgemäßen Katalysatoren können pulverförmig eingesetzt werden. Derartige Katalysatoren weisen vorzugsweise eine mittlere Teilchengröße in einem Bereich von 10 bis 200 µm auf. Pulverförmige Katalysatoren eignen sich vorzugsweise für einen Einsatz in der Wirbelschicht.

Die erfindungsgemäßen Katalysatoren können weiterhin in Form von Katalysatorformkörpern eingesetzt werden. Katalysatorformkörper eignen sich vorzugsweise für einen Einsatz als Festbettkatalysatoren.

Die Formkörper oder Pulver können nach dem Tränken bei erhöhten Temperaturen getrocknet und gegebenenfalls calciniert werden. Die Temperatur beim Trocknen und/oder Calcinieren liegt vorzugsweise in einem Bereich von 50 bis 600 °C, besonders bevorzugt von 100 bis 400 °C.

Die Trocknung der getränkten Katalysatorformkörper kann kontinuierlich oder chargenweise, z. B. in Band- oder Hordenöfen, erfolgen. Die Trocknung kann bei Atmosphärendruck oder vermindertem Druck erfolgen. Des Weiteren kann die Trocknung in einem Gasstrom, z. B. einem Luftstrom oder einem Stickstoffstrom, erfolgen. Im Allgemeinen wird die Trocknung bei Temperaturen von 50 bis 200 °C, vorzugsweise 100 bis 150 °C, durchgeführt.

Die Calcinierung des gegebenenfalls zuvor getrockneten Katalysators erfolgt im Allgemeinen bei Temperaturen von 150 bis 600 °C, vorzugsweise 200 bis 400 °C. Die Calcinierung kann kontinuierlich oder chargenweise, z. B. in Band- oder Hordenöfen, durchgeführt werden. Die Calcinierung kann bei Atmosphärendruck oder vermindertem Druck und/oder in einem Gasstrom erfolgen, z. B. in einem Inertgasstrom, sauerstoffhaltigen Gasstrom, oder wasserstoffhaltigen Gasstrom. Geeignete Inertgase sind z. B. Stickstoff oder Argon. Beim Calcinieren in einer sauerstoffhaltigen Atmosphäre bilden sich aus den Chloriden die Oxide, wie beispielsweise RuO₂. Eine Vorbehandlung mit Wasserstoff oder Wasserstoff enthaltenden Gasen dient im Allgemeinen der Vorreduzierung/Aktivierung des Hydrierkatalysators.

Die Formgebung zu geformten Katalysatorpartikeln kann vor oder nach dem Imprägnieren erfolgen. Als Katalysatorformkörper eignen sich beliebige Formen, bevorzugt sind Tabletten, Ringe, Zylinder, Sterne, Wagenräder oder Kugeln, besonders bevorzugt sind Ringe, Zylinder oder Sternstränge.

Die spezifische Oberfläche des alpha-Aluminiumoxidträgers vor der Metallsalz-Ablagerung liegt im Allgemeinen im Bereich von 0,1 bis 10 m²/g. Alpha-Aluminiumoxid kann durch Erhitzen von gamma-Aluminiumoxid auf Temperaturen oberhalb von 1000 °C hergestellt werden, vorzugsweise wird es so hergestellt. Im Allgemeinen wird 2 bis 24 h lang calciniert.

Für das erfindungsgemäße Verfahren geeignete Rutheniumkatalysatoren und Verfahren zu ihrer Herstellung sind in der WO 2007/023162 beschrieben, auf die hier in vollem Umfang Bezug genommen wird.

In einer bevorzugten Ausführungsform des erfindungsgemäßen Verfahrens wird der Katalysator vor dem Einsatz zur katalytischen Hydrierung von 2-(2-Methyl-prop-1-en-yl)-4-methylen-tetrahydropyran einer Reduktion bei erhöhten Temperaturen in Gegenwart eines wasserstoffhaltigen Gases unterzogen.

Zur Reduktion kann reiner Wasserstoff eingesetzt werden. Bevorzugt wird zur Reduktion ein wasserstoffhaltiges Gas eingesetzt, das Wasserstoff im Gemisch mit wenigstens einem Inertgas enthält. Geeignete Inertgase sind z. B. Stickstoff, Argon oder Helium. Bevorzugt beträgt der Wasserstoffgehalt des wasserstoffhaltigen Gases höchstens 80 Vol.-%, besonders bevorzugt höchstens 60 Vol.-%. Bevorzugt liegt der Wasserstoffgehalt des wasserstoffhaltigen Gases in einem Bereich von 10 bis 60 Vol.-%.

In einer bevorzugten Ausführung wird der reduzierte Katalysator unmittelbar im Anschluss an die Reduktion in eine inerte Flüssigkeit gegeben und darin bis zu seinem Einsatz zur Hydrierung aufbewahrt. Geeignete inerte Flüssigkeiten sind die im Folgenden genannten, zur Durchführung der Hydrierung geeigneten Lösungsmittel. Bevorzugt wird Wasser eingesetzt.

Die Hydrierung des 2-(2-Methyl-prop-1-en-yl)-4-methylen-tetrahydropyrans erfolgt vorzugsweise bei einer Temperatur in einem Bereich von 50 bis 150 °C, besonders bevorzugt von 70 bis 130 °C.

Die Hydrierung des 2-(2-Methyl-prop-1-en-yl)-4-methylen-tetrahydropyrans erfolgt vorzugsweise bei einem absoluten Druck in einem Bereich von 1 bis 50 bar, besonders bevorzugt von 1,5 bis 25 bar, insbesondere von 2 bis 10 bar.

Die Hydrierung des 2-(2-Methyl-prop-1-en-yl)-4-methylen-tetrahydropyrans kann in Gegenwart eines unter den Hydrierbedingungen inerten Lösungsmittels erfolgen. Geeignete Lösungsmittel sind beispielsweise Wasser, Alkohole, wie Methanol, Ethanol, n-Propanol und Isopropanol, aliphatische Kohlenwasserstoffe und Kohlenwasserstoffgemische, wie Hexan, Petrolether und Ligroin, cyclische Ether, wie Tetrahydrofuran, etc.

Der zur Hydrierung eingesetzte Wasserstoff kann in reiner Form oder gewünschtenfalls auch in Form von Gemischen mit anderen, bevorzugt inerten Gasen, wie Stickstoff oder Argon, eingesetzt werden. Bevorzugt setzt man Wasserstoff in unverdünnter Form ein.

Der Umsatz bei der Hydrierung, bezogen auf Rosenoxid, beträgt vorzugsweise wenigstens 90 %, insbesondere wenigstens 94 %.

Die Selektivität bei der Hydrierung, bezogen auf Rosenoxid, beträgt vorzugsweise wenigstens 91 %, besonders bevorzugt wenigstens 95 %. Vielfach können noch höhere Selektivitäten von bis zu 96 % und darüber erzielt werden.

Nach Abtrennung des eingesetzten Katalysators, beispielsweise durch Filtration und gegebenenfalls Abtrennung des eingesetzten Lösungsmittels, vorzugsweise durch Destillation, erhält man ein Reaktionsgemisch, das die diastereomeren Verbindungen der Formeln (IIa) und (IIb) enthält und gegebenenfalls noch weitere Verunreinigungen, unerwünschte Nebenkomponenten oder auch Reste nicht umgesetzten Eduktes enthalten kann.

Eine bevorzugte Ausführung des erfindungsgemäßen Verfahrens ist ein Verfahren, bei dem man
i) ein 2-(2-Methyl-prop-1-en-yl)-4-methylen-tetrahydropyran der Formel (I) 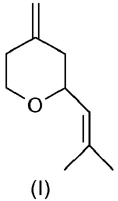 enthaltendes Ausgangsmaterial bereitstellt,
ii) das in Schritt i) bereitgestellte Ausgangsmaterial einer katalytischen Hydrierung in Gegenwart von Wasserstoff und eines heterogenen Katalysators unterzieht, der Ruthenium auf einem Aluminiumoxidträger umfasst, wobei ein cis-2-(2-Methyl-prop-1-en-yl)-4-methyl-tetrahydropyran (IIa) und trans-2-(2-Methyl-prop-1-en-yl)-4-methyl-tetrahydropyran (IIb) 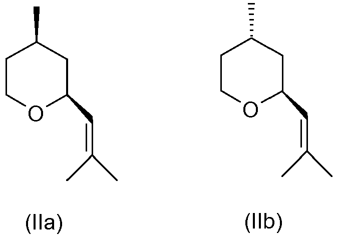 enthaltendes Reaktionsgemisch erhalten wird,
iii) gegebenenfalls die Verbindungen (IIa) und (IIb) von dem in Schritt ii) erhaltene Reaktionsgemisch abtrennt, und
iv) das in Schritt ii) erhaltene Reaktionsgemisch oder die in Schritt iii) abgetrennten Verbindungen (IIa) und (IIb) mit einem stark sauren Ionenaustauscher in Kontakt bringt, wobei die trans-Verbindung (IIb) zumindest teilweise in die cis-Verbindung (IIa) isomerisiert wird.

### Schritt i)

Bevorzugt umfasst die Bereitstellung des 2-(2-Methyl-prop-1-en-yl)-4-methylen-tetra-hydropyrans der Formel (I) enthaltenden Ausgangsgemischs in Schritt i) die Umsetzung von 3-Methyl-but-3-en-1-ol (Isoprenol) der Formel (III) 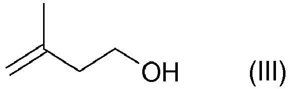 mit 3-Methyl-but-2-en-1-al (Prenal) der Formel (IV) 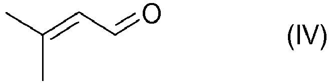 in einer Kondensationsreaktion.

Bevorzugt erfolgt die Umsetzung von 3-Methyl-but-3-en-1-ol (Isoprenol) der Formel (III) mit 3-Methyl-but-2-en-1-al (Prenal) der Formel (IV) in Gegenwart einer Säure und in Gegenwart eines mit Wasser ein Azeotrop bildenden Lösungsmittels.

Vorzugsweise geht man dabei so vor, dass man das bei der vorstehend genannten Umsetzung von 3-Methyl-but-3-en-1-ol der Formel (III) mit 3-Methyl-but-2-en-1-al der Formel (IV) freigesetzte Wasser durch Azeotropdestillation mit dem eingesetzten Lösungsmittel vom Reaktionsgemisch abtrennt. Dabei können sowohl einzelne, mit Wasser ein Azeotrop bildende Lösungsmittel, als auch Gemische verschiedener Lösungsmittel eingesetzt werden. Bevorzugt setzt man dazu solche Lösungsmittel ein, die mit Wasser ein Azeotrop bilden, das einen niedrigeren Siedepunkt als das jeweilige Lösungsmittel bzw. Lösungsmittelgemisch selbst aufweist, vorzugsweise solche, deren azeotroper Siedepunkt im Bereich von etwa 60 °C bis etwa 120 °C, besonders bevorzugt im Bereich von etwa 65 °C bis etwa 90 °C liegt. Bevorzugte Lösungsmittel sind ausgewählt unter Ethanol, Benzol, Tetrachlorkohlenstoff, Essigsäureethylester, Toluol, Chloroform, n-Heptan, Cyclohexan und Methylcyclohexan. Besonders bevorzugte, mit Wasser ein Azeotrop bildende Lösungsmittel sind ausgewählt unter Toluol, Chloroform, n-Heptan, Cyclohexan und Methylcyclohexan. Ganz besonders bevorzugte Lösungsmittel sind Toluol und n-Heptan. Speziell bevorzugt ist Toluol.

Die Abtrennung des bei der Reaktion freigesetzten Wassers durch Azeotropdestillation kann nach dem Fachmann an sich bekannten Verfahren bzw. mit den für diesen Zweck geeigneten Vorrichtungen, wie beispielsweise mit einem Wasserabscheider, durchgeführt werden.

Die im Rahmen der vorstehend genannten Azeotropdestillation einzusetzende Menge an Lösungsmittel kann in einem breiten Bereich gewählt werden und richtet sich in der Regel nach den gewählten Reaktionsbedingungen sowie der zur Wasserabtrennung eingesetzten Vorrichtung. Es hat sich als vorteilhaft erwiesen, das Lösungsmittel in einem auf die Gesamtmenge der eingesetzten Edukte 3-Methyl-but-3-en-l-ol (III) und 3-Metyhl-but-2-en-1-al (IV) bezogenem Mengenverhältnis von etwa 1 : 1 bis etwa 2 : 1, besonders bevorzugt von etwa 1 : 1 bis etwa 1,5 : 1, einzusetzen. Das Lösungsmittel kann nach Durchführung der Reaktion in der Regel gut abgetrennt und im Rahmen weiterer Umsetzungen wieder eingesetzt werden.

Die Bereitstellung von Dehydrorosenoxid der Formel (I) durch Umsetzung von 3-Methyl-but-3-en-1-ol (III) und 3-Methyl-but-2-en-1-al (IV) wird vorzugsweise in Gegenwart einer Säure durchgeführt. Geeignete Säuren sind sowohl organische als auch anorganische Säuren, wie beispielsweise p-Toluolsulfonsäure, Trifluoressigsäure oder Alkalihydrogensulfate. In einer bevorzugten Ausführungsform führt man die Umsetzung von 3-Methylbut-3-en-1-ol (III) mit 3-Methyl-but-2-en-1-al (IV) in Gegenwart eines Alkalihydrogensulfates, wie beispielsweise Natriumhydrogensulfat oder Kaliumhydrogensulfat, durch. Bevorzugt ist Natriumhydrogensulfat.

Die gewählte Säure wird bevorzugt in katalytischen Mengen eingesetzt, üblicherweise bezogen auf die Gesamtmenge der umzusetzenden Ausgangsstoffe 3-Methyl-but-3-en-1-ol (III) und 3-Methyl-but-2-en-1-al (IV), in einer Menge von etwa 0,01 bis etwa 1 Gew.-%.

Die Reaktion zur Herstellung von Dehydrorosenoxid durch Kondensation von Isoprenol mit Prenal wird in der Regel und in Abhängigkeit vom gewählten Lösungsmittel bzw. Lösungsmittelgemisch und der gewählten Säure bei Temperaturen im Bereich von etwa 60 °C bis 150 °C, bevorzugt im Bereich von etwa 70 °C bis 120 °C durchgeführt und ist dann in der Regel rasch, oft nach etwa 24 h oder auch früher, weitgehend abgeschlossen. Das erhaltene Reaktionsgemisch kann nach dem Fachmann bekannten Verfahren, beispielsweise durch extraktive Verfahren, gegebenenfalls nach Neutralisation der eingesetzten Säure, aufgearbeitet werden. Das so als Rohprodukt erhaltene Dehydrorosenoxid der Formel (I) kann im Anschluss weiter aufgereinigt werden, beispielsweise durch Chromatographie oder bevorzugt durch (fraktionierte) Destillation, wobei insbesondere das als Nebenprodukt üblicherweise anfallende Neroloxid sowie weitere hochsiedende Nebenkomponenten abgetrennt werden können.

### Schritt ii)

Bezüglich geeigneter und bevorzugter Ausführungsformen zu Schritt ii) wird auf die vorherigen Ausführungen zur katalytischen Hydrierung von 2-(2-Methyl-prop-1-en-yl)-4-methylen-tetrahydropyran in Gegenwart von Wasserstoff und eines heterogenen Katalysators, der Ruthenium auf einem Aluminiumoxidträger umfasst, in vollem Umfang Bezug genommen.

### Schritt iii)

Gemäß dem optional durchzuführenden Verfahrensschritt iii) des erfindungsgemäßen Verfahrens kann man gewünschtenfalls eine Abtrennung der Verbindungen der Formel (IIa) und (IIb) von dem gemäß Schritt ii) erhaltenen Reaktionsgemisch durchführen. Dazu stehen die dem Fachmann als geeignet erscheinenden Methoden der Stofftrennung, wie beispielsweise chromatographische Verfahren oder bevorzugt Destillation, zur Verfügung. Als geeignete Destillationsvorrichtungen seien beispielsweise Vorrichtungen zur Kurzwegdestillation, wie beispielsweise Dünnschichtverdampfer, genannt oder auch gefüllte oder gepackte Kolonnen sowie Bodenkolonnen.

### Schritt iv)

Das auf diese Weise gemäß Verfahrenschritt ii) oder nach Aufreinigung gemäß dem optionalen Verfahrensschritt iii) erhaltene Gemisch der Verbindungen der Formel (IIa) und (IIb) wird anschließend im Verfahrensschritt iv) mit einem stark sauren Kationenaustauscher in Kontakt gebracht. Dabei erfolgt eine zumindest teilweise Isomerisierung der trans-Verbindung der Formel (IIb) zur cis-Verbindung der Formel (IIa).

Nach Durchführung des Verfahrensschritts iv) erhält man Gemische der Verbindungen der Formeln (IIa) und (IIb), die einen höheren Gehalt der gewünschten Verbindung der Formel (IIa) aufweisen als die zunächst durch die Verfahrensschritte ii) bzw. iii) erhaltenen Gemische. Man erhält auf diese Weise bevorzugt die vorstehend genannten Diastereomeren-angereicherten Gemische von cis-2-(2-Methyl-prop-1-en-yl)-4-methyl-tetrahydropyran der Formel (IIa) und trans-2-(2-Methyl-prop-1-en-yl)-4-methyltetra-hydropyran der Formel (IIb), enthaltend, bezogen auf die Menge des Isomerengemisches, mindestens 70 %, bevorzugt mindestens 90 % und besonders bevorzugt 90 bis 98 % cis-2-(2-Methyl-prop-1-en-yl)-4-methyl-tetrahydropyran der Formel (I) und höchstens 30 %, bevorzugt höchstens 10 % und besonders bevorzugt 2 bis 10 % trans-2-(2-Methyl-prop-1-en-yl)-4-methyl-tetrahydropyran der Formel (IIb).

Die Isomerisierung gemäß Verfahrensschritt iv) des erfindungsgemäßen Verfahrens erfolgt in Gegenwart eines stark sauren Ionentauschers, d. h. eines stark sauren Kationentauschers, wie beispielsweise Lewatit® S100, Lewatit® SP1 12, Lewatit® S115, Lewatit® SP1080, Lewatit® SC102, Lewatit® SPC1 18, Lewatit® CNP 80, Lewatit® HD 5, Amberlite® IR 120, Amberlite® IR200, Amberlyst® 15, Bay. KAT. K 2431, Bay. KAT. K 2621 , Dowex® 50, Permutit® RS, Wofatit® KPS 200, Duolite® C-3, Duolite® C-10, Duolite® C-25, Wofatit® F, Wofatit® D, Wofatit® P, Zeoxex (Zeokarb H), Nalcite HCR, Nalcite HGR, Nalcite HDR, Permutit® Q und Permutit® RS, Serdrolit® rot. Die gewählten stark sauren Kationentauscher können auch in Form von Gemischen zweier oder mehrerer verschiedener Kationentauscher eingesetzt werden. Bevorzugt setzt man die Kationenaustauscher Lewatit® SP1 12 und/oder Amberlyst® 15 ein.

Im Rahmen einer bevorzugten Ausführungsform des erfindungsgemäßen Verfahrens setzt man den gewählten Kationentauscher in Form eines Festbetts ein, über das das umzusetzende, aus Verfahrensschritt ii) bzw. iii) erhaltene Diastereomerengemisch als solches oder in Form einer Lösung in einem geeigneten, unter den Reaktionsbedingungen inerten, Lösungsmittel geleitet wird. Bevorzugt bringt man das zu isomerisierende Gemisch in unverdünnter Form mit dem gewählten, stark sauren Kationentauscher in Kontakt. Dabei kann das Festbett beispielsweise in Form einer Schüttung des gewählten Kationentauschers in einem Reaktionsrohr ausgestaltet sein, wobei das zu isomerisierende Gemisch durch das so befüllte Reaktionsrohr geleitet wird. Dazu können die Reaktoren in allen dem Fachmann geeignet erscheinenden Betriebs- bzw. Fahrweisen betrieben werden, wie beispielsweise in Sumpffahrweise oder erfindungsgemäß bevorzugt in Rieselfahrweise, wobei das zu isomerisierende Gemisch auf eine Schüttung des gewählten Kationentauschers gerieselt wird.

Auf diese Weise ist auch die erfindungsgemäß im Rahmen von Verfahrensschritt iv) bevorzugte kontinuierliche Reaktionsführung möglich. Im Rahmen einer bevorzugten Ausführungsform führt man daher Verfahrensschritt iv) kontinuierlich durch. Dabei wird das zu isomerisierende, die Verbindungen der Formel (IIa) und (IIb) enthaltende Gemisch kontinuierlich dem Kationentauscher zugeführt, beispielsweise durch Eintragen in einen mit Kationentauscher befüllten Reaktor und kontinuierlich von demselben wieder abgetrennt, beispielsweise durch Austragen des isomerisierten Gemisches aus dem Reaktor.

Das zu isomerisierende Gemisch der Verbindungen der Formeln (IIa) und (IIb) kann auch mehrmals hintereinander mit dem gewählten stark sauren Kationentauscher oder auch mit verschiedenen stark sauren Kationentauschern in Kontakt gebracht werden, beispielsweise durch Rückführung des aus dem wie vorstehend beschriebenen Festbettreaktor ausgetragenen, Diastereomeren-angereicherten Isomerengemischs in den gleichen Reaktor. Es ist auch möglich, mehrere derartige Reaktoren, die gewünschtenfalls auch mit verschiedenen Kationentauschern befüllt sein können, hintereinander zu durchlaufen, um so zu dem gewünschten, wie vorstehend beschriebenen Diastereomerenverhältnis zu gelangen.

Die Isomerisierung gemäß Verfahrensschritt iv) wird üblicherweise bei Temperaturen von etwa 0 °C bis etwa 100 °C, bevorzugt bei etwa 20 bis etwa 80 °C, durchgeführt.

Die folgenden Beispiele dienen der Verdeutlichung der Erfindung, ohne sie in irgendeiner Weise zu beschränken.

### Beispiele

### Beispiel 1: Herstellung von Dehydrorosenoxid (analog zu WO 2009/077550, Beispiel 1)

In einem mit Rührer, Wasserauskreiser, Kühler und einer Dosierpumpe versehenen Reaktionsgefäß mit einem Volumen von 5 I wurden 2000 g Toluol und 1,5 g NaHSO₄ (als 10%ige wässrige Lösung) vorgelegt und 7,67 mol (660 g) 3-Methyl-but-3-en-1-ol und 7,67 mol (643,5 g) 3-Methyl-but-2-en-1-al innerhalb 16 h bei 110 bis 115 °C zudosiert. Das Wasser wurde kontinuierlich aus dem Reaktionsgemisch mit Toluol ausgekreist und das Toluol zurückgeführt. In Anschluss wurde das Reaktionsgemisch weitere 5,5 h bei 115 °C gerührt. Das erhaltene Reaktionsgemisch wurde anschließend mit 278 g 2%iger NaOH-Lösung gewaschen. Das Toluol wurde bei einem Druck von 200 mbar über einer 30 cm lange, mit Raschigringen gefüllte, Kolonne abdestilliert. Der Umsatz zu Dehydrorosenoxid (DHR) betrug 62,7 % der Theorie. Abschließend wurde das DHR durch Destillation von Neroloxid und hochsiedenden Nebenkomponenten getrennt und mit einer Reinheit von > 99 % erhalten.

### Beispiel 2 (erfindungsgemäß):

Zunächst wurde gemäß Beispiel 1 der WO 2007/023161 ein RuO₂-Katalysator auf einem alpha-Al₂O₃-Träger hergestellt. Dazu wurde ein pulverförmiger gamma-Aluminiumoxid-Träger der Fa. Sasol (Puralox (R) SCCa 30/170) zunächst in alpha-Al₂O₃ umgewandelt. Der Träger besteht aus Partikeln mit einem mittleren Teilchendurchmesser von ca. 50 µm. 2000 g des Puralox (R) SCCa 30/170 wurden bei 1200 bis 1300 °C 5 h lang getempert. 1500 g des erhaltenen Trägers wurden mit einer wässrigen RuCl₃-Hydrat-Lösung (55,56 g RuCl₃-Hydrat, entsprechend 41,8 Gew.-% Ru in 480 g Wasser) getränkt. Die Wasseraufnahme des Trägers betrug ca. 0,38 ml/g. Nach der Tränkung mit 90 % Wasseraufnahme wurde der imprägnierte Träger 6 h lang bei 120 °C getrocknet und anschließend 2 h bei 350 °C calciniert. Der so hergestellte Katalysator enthielt ca. 2 % RuO₂ auf aipha-Al₂O₃. Der Katalysator wurde dann zunächst bei 200 °C in einem verdünnten Wasserstoffstrom (50 Vol.-% H₂, 50 Vol.-% N₂) reduziert und nach dem Abkühlen mittels Durchspülen mit entgastem Wasser in ein Vorlagegefäß gefüllt.

In einen 500 ml Büchi-Laborautoklaven wurden 7,3 g (= 0,11 g Ru) dieses Katalysators (berechnet auf Trockenbasis) und 220 g Dehydrorosenoxid aus Beispiel 1 gefüllt und bei 4 bar Wasserstoffdruck absolut und einer Temperatur von 70 °C zu Rosenoxid umgesetzt. Nach 4 Stunden Reaktionszeit betrug der Umsatz 94 % und die Selektivität zu Rosenoxid 96 %.

### Beispiel 3 (Vergleich): (analog zu WO 2009/077550, Beispiel 2)

Es wurde ein Katalysator gemäß Beispiel 1 der EP 0071787 eingesetzt, der 5 Gew.-% Ru und 1 Gew.-% Fe auf einem Aktivkohleträger enthält.

In einen 500 ml Büchi-Laborautoklaven wurden 2,2 g (= 0,11 g Ru) dieses Katalysators (berechnet auf Trockenbasis) und 220 g Dehydrorosenoxid aus Beispiel 1 gefüllt und bei 4 bar Wasserstoffdruck absolut und einer Temperatur von 70 °C zu Rosenoxid umgesetzt. Nach 5 Stunden Reaktionszeit betrug der Umsatz 93 % und die Selektivität zu Rosenoxid 90 %.

## Patentansprüche

1. Verfahren zur Herstellung einer an cis-2-(2-Methyl-prop-1-en-yl)-4-methyl-tetrahydropyran angereicherten Zusammensetzung, umfassend die katalytische Hydrierung von 2-(2-Methyl-prop-1-en-yl)-4-methylen-tetrahydropyran in Gegenwart von Wasserstoff und eines heterogenen Katalysators, der Ruthenium auf einem Aluminiumoxidträger umfasst.

2. Verfahren nach Anspruch 1, wobei der Aluminiumoxidträger zu wenigstens 80 Gew.-%, bezogen auf das Gesamtgewicht des Trägermaterials, aus Aluminiumoxid besteht.

3. Verfahren nach Anspruch 1 oder 2, wobei der Aluminiumoxidträger zu wenigstens 90 Gew.-%, bezogen auf das Gesamtgewicht des Trägermaterials, aus Aluminiumoxid besteht.

4. Verfahren nach einem der vorhergehenden Ansprüche, wobei der Aluminiumoxidträger zu wenigstens 96 Gew.-%, bezogen auf das Gesamtgewicht des Trägermaterials, aus Aluminiumoxid besteht.

5. Verfahren nach einem der vorhergehenden Ansprüche, wobei der Aluminiumoxidträger zu mindestens 80 Gew.-% aus alpha-Aluminiumoxid, bezogen auf den gesamten Aluminiumoxidanteil des Trägers, besteht.

6. Verfahren nach einem der vorhergehenden Ansprüche, wobei der Aluminiumoxidträger zu mindestens 98 Gew.-% aus alpha-Aluminiumoxid, bezogen auf den gesamten Aluminiumoxidanteil des Trägers, besteht.

7. Verfahren nach einem der vorhergehenden Ansprüche, wobei ein Katalysator eingesetzt wird, der 0,001 bis 10 Gew.-% Ruthenium, bezogen auf das Gesamtgewicht des Katalysators, enthält.

8. Verfahren nach einem der vorhergehenden Ansprüche, wobei ein Katalysator eingesetzt wird, der 1 bis 3 Gew.-% Ruthenium, bezogen auf das Gesamtgewicht des Katalysators, enthält.

9. Verfahren nach einem der vorhergehenden Ansprüche, wobei ein Katalysator eingesetzt wird, der
a) 0,001 bis 10 Gew.-% Ruthenium,
b) 0 bis 5 Gew.-% eines oder mehrerer Erdalkalimetalle,
c) 0 bis 5 Gew.-% eines oder mehrerer Alkalimetalle,
d) 0 bis 10 Gew.-% eines oder mehrerer Seltenerdmetalle,
e) 0 bis 10 Gew.-% eines oder mehrerer weiterer Metalle, ausgewählt aus der Gruppe bestehend aus Kupfer, Gold, Palladium, Platin, Osmium, Iridium, Silber und Rhenium,
jeweils bezogen auf das Gesamtgewicht des Katalysators, enthält.

10. Verfahren nach einem der vorhergehenden Ansprüche, wobei ein Katalysator eingesetzt wird, der als alleinige Metallkomponente 1 bis 3 Gew.- % Ruthenium in Form von RuO₂ auf einem alpha-Aluminiumoxidträger enthält.

11. Verfahren nach einem der vorhergehenden Ansprüche, wobei der Katalysator vor dem Einsatz zur katalytischen Hydrierung von 2-(2-Methyl-prop-1-en-yl)-4-methylen-tetrahydropyran einer Reduktion bei erhöhten Temperaturen in Gegenwart eines wasserstoffhaltigen Gases unterzogen wird.

12. Verfahren nach einem der vorhergehenden Ansprüche, bei dem man
i) ein 2-(2-Methyl-prop-1-en-yl)-4-methylen-tetrahydropyran der Formel (I) 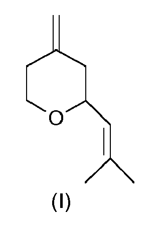 enthaltendes Ausgangsmaterial bereitstellt,
ii) das in Schritt i) bereitgestellte Ausgangsmaterial einer katalytischen Hydrierung in Gegenwart von Wasserstoff und eines heterogenen Katalysators unterzieht, der Ruthenium auf einem Aluminiumoxidträger umfasst, wobei ein cis-2-(2-Methyl-prop-1-en-yl)-4-methyl-tetrahydropyran (IIa) und trans-2-(2-Methyl-prop-1-en-yl)-4-methyl-tetrahydropyran (IIb) 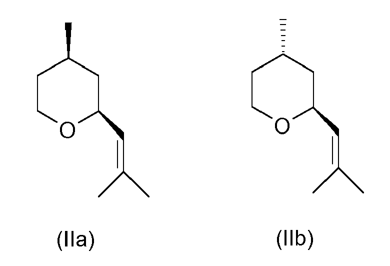 enthaltendes Reaktionsgemisch erhalten wird,
iii) gegebenenfalls die Verbindungen (IIa) und (IIb) von dem in Schritt ii) erhaltene Reaktionsgemisch abtrennt, und
iv) das in Schritt ii) erhaltene Reaktionsgemisch oder die in Schritt iii) abgetrennten Verbindungen (IIa) und (IIb) mit einem stark sauren Ionenaustauscher in Kontakt bringt, wobei die trans-Verbindung (IIb) zumindest teilweise in die cis-Verbindung (IIa) isomerisiert wird.

13. Verfahren nach Anspruch 12, wobei die Bereitstellung des 2-(2-Methyl-prop-1-en-yl)-4-methylen-tetrahydropyran der Formel (I) enthaltenden Ausgangsgemischs in Schritt i) die Umsetzung von 3-Methyl-but-3-en-1-ol der Formel (III) 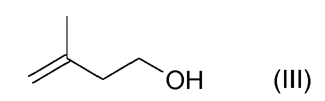 mit 3-Methyl-but-2-en-1-al der Formel (IV) 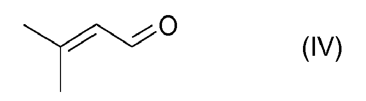 in einer Kondensationsreaktion umfasst.

14. Verfahren nach Anspruch 13, wobei die Umsetzung von 3-Methyl-but-3-en-1-ol der Formel (III) mit 3-Methyl-but-2-en-1-al der Formel (IV) in Gegenwart einer Säure und in Gegenwart eines mit Wasser ein Azeotrop bildenden Lösungsmittels erfolgt.

15. Verfahren nach Anspruch 14, wobei das bei der Umsetzung von 3-Methyl-but-3-en-1-ol der Formel (III) mit 3-Methyl-but-2-en-1-al der Formel (IV) freigesetzte Wasser durch Azeotropdestillation mit dem eingesetzten Lösungsmittel vom Reaktionsgemisch abgetrennt wird.

## Claims

1. A process for the preparation of a composition enriched in cis-2-(2-methylprop-1-enyl)-4-methyltetrahydropyran comprising the catalytic hydrogenation of 2-(2-methylprop-1-enyl)-4-methylenetetrahydropyran in the presence of hydrogen and a heterogeneous catalyst comprising ruthenium on an aluminum oxide support.

2. The process according to claim 1, wherein the aluminum oxide support consists to at least 80% by weight, based on the total weight of the support material, of aluminum oxide.

3. The process according to claim 1 or 2, wherein the aluminum oxide support consists to at least 90% by weight, based on the total weight of the support material, of aluminum oxide.

4. The process according to any one of the preceding claims, wherein the aluminum oxide support consists to at least 96% by weight, based on the total weight of the support material, of aluminum oxide.

5. The process according to any one of the preceding claims, wherein the aluminum oxide support consists to at least 80% by weight of alpha-aluminum oxide, based on the total aluminum oxide fraction of the support.

6. The process according to any one of the preceding claims, wherein the aluminum oxide support consists of at least 98% by weight of alpha-aluminum oxide, based on the total aluminum oxide fraction of the support.

7. The process according to any one of the preceding claims, wherein a catalyst is used which comprises 0.001 to 10% by weight of ruthenium, based on the total weight of the catalyst.

8. The process according to any one of the preceding claims, wherein a catalyst is used which comprises 1 to 3% by weight of ruthenium based on the total weight of the catalyst.

9. The process according to any one of the preceding claims, wherein a catalyst is used which comprises
a) 0.001 to 10% by weight of ruthenium,
b) 0 to 5% by weight of one or more alkaline earth metals,
c) 0 to 5% by weight of one or more alkali metals,
d) 0 to 10% by weight of one or more rare earth metals,
e) 0 to 10% by weight of one or more further metals selected from the group consisting of copper, gold, palladium, platinum, osmium, iridium, silver and rhenium,
in each case based on the total weight of the catalyst.

10. The process according to any one of the preceding claims, wherein a catalyst is used which comprises, as the sole metal component, 1 to 3% by weight of ruthenium in the form of RuO₂ on an alpha-aluminum oxide support.

11. The process according to any one of the preceding claims, wherein the catalyst is subjected, prior to being used for the catalytic hydrogenation of 2-(2-methylprop-1-enyl)-4-methylenetetrahydropyran, to a reduction at elevated temperatures in the presence of a hydrogen-containing gas.

12. The process according to any one of the preceding claims, in which
i) a starting material comprising 2-(2-methylprop-1-enyl)-4-methylenetetrahydropyran of the formula (I) 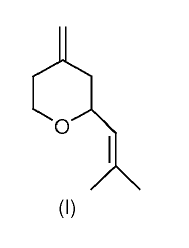 is prepared,
ii) the starting material prepared in step i) is subjected to a catalytic hydrogenation in the presence of hydrogen and a heterogeneous catalyst comprising ruthenium on an aluminum oxide support, giving a reaction mixture comprising cis-2-(2-methylprop-1-enyl)-4-methyltetrahydropyran (IIa) and trans-2-(2-methylprop-1-enyl)-4-methyltetrahydropyran (IIb) 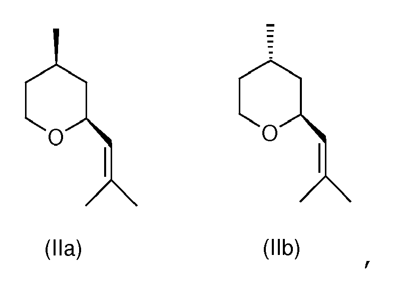
iii) optionally the compounds (IIa) and (IIb) are separated off from the reaction mixture obtained in step ii), and
iv) the reaction mixture obtained in step ii) or the compounds (IIa) and (IIb) separated off in step iii) are brought into contact with a strong acidic ion exchanger, the transcompound (IIb) being isomerized at least partially into the cis compound (IIa).

13. The process according to claim 12, wherein the preparation of the starting mixture comprising 2-(2-methylprop-1-enyl)-4-methylenetetrahydropyran of the formula (I) in step i) comprises the reaction of 3-methylbut-3-en-1-ol of the formula (III) 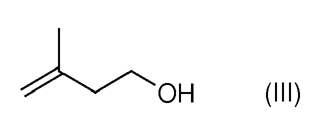 with 3-methylbut-2-en-1-al of the formula (IV) 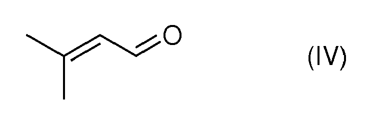 in a condensation reaction.

14. The process according to claim 13, wherein the reaction of 3-methylbut-3-en-1-ol of the formula (III) with 3-methylbut-2-en-1-al of the formula IV takes place in the presence of an acid and a solvent which forms an azeotrope with water.

15. The process according to claim 14, wherein the water released during the reaction of 3-methylbut-3-en-1-ol of the formula (III) with 3-methylbut-2-en-1-al of the formula (IV) is separated from the reaction mixture by azeotropic distillation with the solvent used.

## Revendications

1. Procédé pour la préparation d'une composition enrichie en cis-2-(2-méthylprop-1-ényl)-4-méthyltétrahydropyrane, comprenant l'hydrogénation catalytique de 2-(2-méthylprop-1-ényl)-4-méthylènetétrahydropyrane en présence d'hydrogène et d'un catalyseur hétérogène, qui comprend du ruthénium sur un support d'oxyde d'aluminium.

2. Procédé selon la revendication 1, le support d'oxyde d'aluminium étant constitué, à raison d'au moins 80% en poids, par rapport au poids total du matériau support, d'oxyde d'aluminium.

3. Procédé selon la revendication 1 ou 2, le support d'oxyde d'aluminium étant constitué, à raison d'au moins 90% en poids, par rapport au poids total du matériau support, d'oxyde d'aluminium.

4. Procédé selon l'une quelconque des revendications précédentes, le support d'oxyde d'aluminium étant constitué, à raison d'au moins 96% en poids, par rapport au poids total du matériau support, d'oxyde d'aluminium.

5. Procédé selon l'une quelconque des revendications précédentes, le support d'oxyde d'aluminium étant constitué, à raison d'au moins 80% en poids, par rapport à la proportion totale d'oxyde d'aluminium du support, d'alpha-oxyde d'aluminium.

6. Procédé selon l'une quelconque des revendications précédentes, le support d'oxyde d'aluminium étant constitué, à raison d'au moins 98% en poids, par rapport à la proportion totale d'oxyde d'aluminium du support, d'alpha-oxyde d'aluminium.

7. Procédé selon l'une quelconque des revendications précédentes, un catalyseur contenant 0,001 à 10% en poids de ruthénium, par rapport au poids total du catalyseur, étant utilisé.

8. Procédé selon l'une quelconque des revendications précédentes, un catalyseur contenant 1 à 3% en poids de ruthénium, par rapport au poids total du catalyseur, étant utilisé.

9. Procédé selon l'une quelconque des revendications précédentes, un catalyseur étant utilisé, qui contient
a) 0,001 à 10% en poids de ruthénium,
b) 0 à 5% en poids d'un ou de plusieurs métaux alcalino-terreux,
c) 0 à 5% en poids d'un ou de plusieurs métaux alcalins,
d) 0 à 10% en poids d'un ou de plusieurs métaux des terres rares,
e) 0 à 10% en poids d'un ou de plusieurs autres métaux, choisis dans le groupe constitué par le cuivre, l'or, le palladium, le platine, l'osmium, l'iridium, l'argent et le rhénium,
à chaque fois par rapport au poids total du catalyseur.

10. Procédé selon l'une quelconque des revendications précédentes, un catalyseur contenant, comme composant métallique unique, 1 à 3% en poids de ruthénium sous forme de RuO₂ sur un support d'alpha-oxyde d'aluminium, étant utilisé.

11. Procédé selon l'une quelconque des revendications précédentes, le catalyseur étant soumis, avant l'utilisation pour l'hydrogénation catalytique de 2-(2-méthylprop-1-ényl)-4-méthylènetétrahydropyrane, à une réduction à des températures augmentées en présence d'un gaz contenant de l'hydrogène.

12. Procédé selon l'une quelconque des revendications précédentes, dans lequel
i) on prépare un matériau de départ contenant du 2-(2-méthylprop-1-ényl)-4-méthylènetétrahydropyrane de formule (I), 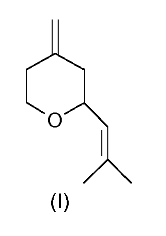
ii) on soumet le matériau de départ préparé dans l'étape i) à une hydrogénation catalytique en présence d'hydrogène et d'un catalyseur hétérogène, qui comprend du ruthénium sur un support d'oxyde d'aluminium, un mélange réactionnel contenant du cis-2-(2-méthylprop-1-ényl)-4-méthyltétrahydropyrane (IIa) et du trans-2-(2-méthylprop-1-ényl)-4-méthyltétrahydropyrane (IIb) étant obtenu, 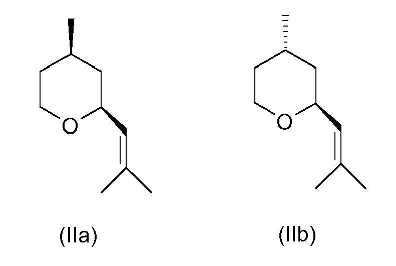
iii) on sépare le cas échéant les composés (IIa) et (IIb) du mélange réactionnel obtenu dans l'étape ii) et
iv) on met en contact le mélange réactionnel obtenu dans l'étape ii) ou les composés (IIa) et (IIb) séparés dans l'étape iii) avec un échangeur ionique fortement acide, le composé trans (IIb) étant isomérisé au moins partiellement en composé cis (IIa).

13. Procédé selon la revendication 12, la préparation du mélange de départ contenant le 2-(2-méthylprop-1-ényl)-4-méthylènetétrahydropyrane de formule (I) dans l'étape i) comprenant la transformation de 3-méthylbut-3-én-1-ol de formule (III) 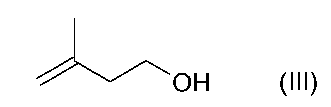 avec du 3-méthylbut-2-én-1-al de formule (IV) 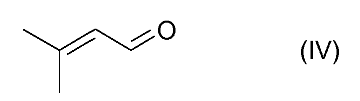 dans une réaction de condensation.

14. Procédé selon la revendication 13, la transformation du 3-méthylbut-3-én-1-ol de formule (III) avec du 3-méthylbut-2-én-1-al de formule (IV) ayant lieu en présence d'un acide et en présence d'un solvant formant un azéotrope avec l'eau.

15. Procédé selon la revendication 14, l'eau libérée lors de la transformation du 3-méthylbut-3-én-1-ol de formule (III) avec du 3-méthylbut-2-én-1-al de formule (IV) étant séparée du mélange réactionnel par distillation azéotropique avec le solvant utilisé.
